# EUROPÄISCHE PATENTSCHRIFT

(11) **EP 0 527 217 B1**
(45) Veröffentlichungstag und Bekanntmachung des Hinweises auf die Patenterteilung: **07.05.1997**
(21) Anmeldenummer: 92905637.2
(22) Anmeldetag: 27.02.1992
(51) Int. Cl.: G01N 33/52

(54) **Verfahren zur Herstellung eines selbsttragenden Testfeldmaterials und solches Testfeldmaterial**
Process for the manufacture of a self-supporting test field material and said material
Procédé de fabrication d'un matériau autoporteur pour une zone de test et ledit matériau

(30) Priorität: 28.02.1991 DE 4106293; 24.06.1991 DE 4120823
(43) Veröffentlichungstag der Anmeldung: 17.02.1993
(73) Patentinhaber: BOEHRINGER MANNHEIM GMBH, 68298 Mannheim (DE)
(72) Erfinder: PLESCH, Winfried, D-6915 Dossenheim (DE); MOSOIU, Dan, D-6703 Limburgerhof (DE); GÖTSCHEL, Hans, D-6800 Mannheim 1 (DE)
(74) Vertreter: Pfeifer, Hans-Peter, Dr., Dr. H.-P. Pfeifer Dr. P. Jany, Patentanwälte
(86) Internationale Anmeldenummer: DE9200160
(87) Internationale Veröffentlichungsnummer: WO9215880

(56) Entgegenhaltungen:
- EP-A- 0 073 056
- EP-A- 0 246 505
- EP-A- 0 297 390
- EP-A- 0 302 287

## Beschreibung

Die Erfindung betrifft ein Verfahren zur Herstellung eines selbsttragenden Testfeldmaterials sehr geringer Schichtdicke und ein derartiges Testfeldmaterial.

Zur qualitativen und/oder quantitativen analytischen Bestimmung von Bestandteilen von Körperflüssigkeiten, insbesondere Blut und Urin, werden zunehmend sogenannte trägergebundene Tests verwendet. Bei diesen ist ein Reagenzsystem in mindestens einem aus einer oder mehreren Schichten bestehenden Testfeld eines Testträgers (in der englischsprachigen Literatur vielfach auch als "solid reagent analysis element" bezeichnet) eingebettet, das mit der Probe in Kontakt gebracht wird. Die Reaktion von Probe und Reagenz führt zu einer optisch oder in sonstiger Weise nachweisbaren Veränderung, welche visuell oder mit Hilfe eines Gerätes (meist reflexionsphotometrisch) ausgewertet werden kann.

Die Schichtdicke der verwendeten Testfeldmaterialien ist von erheblicher Bedeutung für die Qualität der Analyse. Im allgemeinen wird eine möglichst geringe Schichtdicke angestrebt. Dadurch läßt sich mit einer geringen Menge an Reagenzien eine intensive Farbbildung erreichen, die für die Genauigkeit der Analyse von großer Bedeutung ist. Außerdem wird durch eine geringe Schichtdicke der Bedarf an Probenflüssigkeit reduziert. Dies ist vor allem dann wichtig, wenn bei modernen analytischen Verfahren mehrere Analysen aus einem einzigen (durch einen Stich in einen Finger gewonnenen) Blutstropfen durchgeführt werden sollen.

Aus diesem Grund werden häufig dünne Reagenzfilme verwendet, die teilweise eine Dicke von weniger als 0,1 mm haben. Sie werden in einem schichtbildenden Verfahren durch Auftragen einer filmbildenden Masse (Reagenzfilmmasse) auf eine Tragschicht (meist eine durchsichtige Kunststoffolie) hergestellt. Die Reagenzfilmmasse basiert auf einer Dispersion oder Lösung eines polymeren Filmbildners und enthält die in der jeweiligen Testschicht benötigten Reagenzien.

Im allgemeinen werden solche Reagenzfilme zusammen mit dem Trägermaterial in dem Testträger konfektioniert (beispielsweise in einen Rahmen eingefaßt oder auf eine streifenförmige Basisschicht aufgeklebt). Es ist auch schon beschrieben worden, die Filmschicht von der Tragfolie, auf der sie gebildet wurde, wieder abzuziehen. Dies ist bei dünnen Filmen aber in der Praxis nicht durchführbar.

Es fehlt daher an einem Testmaterial sehr geringer Stärke (weniger als 0,1 mm), welches als selbsttragendes Testfeldmaterial geeignet ist, d.h. ohne vollflächige Verbindung mit einer Tragschicht einsetzbar ist.

Ein Testfeldmaterial im Sinne der vorliegenden Erfindung ist jedes dünne Flächengebilde, welches als selbständiger Bestandteil in das Testfeld eines Testträgers integriert werden kann. Das Testfeld kann ausschließlich aus diesem Testfeldmaterial bestehen. Es können auch mehrere erfindungsgemäße Testfeldmaterialien oder Kombinationen mit anderen in der Testträgertechnik üblichen Schichtmaterialien, wie beispielsweise Papiere, Kunststoffmembranen oder Fasermaterialien verwendet werden.

Der Erfindung liegt die Aufgabe zugrunde, ein Testfeldmaterial zur Verfügung zu stellen, welches bei sehr geringer Schichtdicke selbsttragende Eigenschaften hat und die zusätzlichen schwierigen Anforderungen auf dem Gebiet der Analyse von Körperflüssigkeiten erfüllt.

Zur Lösung dieser Aufgabe wird ein Verfahren gemäß Anspruch 1 vorgeschlagen.

Der Begriff "Faserverbundstruktur" bezeichnet jede Form des Verbundes von Fasern oder Fäden zu einer dünnen offenporigen Schicht. Beispiele sind Gewebe, Gewirke und Vliese. Besonders bewährt hat sich die Verwendung eines einlagigen Gewebes oder Gewirkes, welches vorzugsweise aus monophilen Fäden besteht. Die Porenweite der Faserverbundstruktur sollte im Mittel zwischen 0,02 mm und 0,08 mm, vorzugsweise zwischen 0,03 mm und 0,05 mm liegen.

Die Zusammensetzung der Reagenzfilmmasse ist im wesentlichen von der Funktion des erfindungsgemäßen Testfeldmaterials in dem jeweiligen Testträger abhängig. Sie enthält neben einem dispergierten oder gelösten polymeren Filmbildner die für den jeweiligen Test erforderlichen Reagenzbestandteile (z.B. Enzyme, Substrate und Pufferreagenzien) und Hilfsstoffe wie beispielsweise Pigment oder Öffnerbestandteile. In jedem Fall sollte die Viskosität der Reagenzfilmmasse zum Zeitpunkt des Imprägnierens des Trägermaterials zwischen 10 und 300 mPa.s, bevorzugt zwischen 20 und 200 mPa.s liegen.

Gegenstand der Erfindung ist auch ein Testfeldmaterial für einen Testträger zur Bestimmung eines Analyten in einer Körperflüssigkeit, das eine offenporige Faserverbundstruktur aufweist, in die ein Reagenzfilmmaterial derartig eingebettet ist, daß sie deren Poren überspannt und somit schließt, wobei das Reagenzfilmmaterial aus einer filmbildenden zähflüssigen Reagenzfilmmasse gebildet ist, welche in die Faserverbundstruktur eingepreßt und beidseitig glattgestrichen ist, so daß das Reagenzfilmmaterial im wesentlichen symmetrisch über die Schichtdicke des Testfeldmaterials verteilt ist.

Die Verwendung eines Verbundes einer Faserschicht mit einem Reagenzfilm ist aus den US-Patentschriften 4 292 272 und 4 604 264 bekannt. Im erstgenannten Fall wird auf eine Kunststoffolie eine filmbildende Masse aufgebracht und das Gewebe in die noch feuchte Masse eingedrückt. Das Gewebe dient dabei in erster Linie zur Ausbreitung der Probe über die Testfeldoberfläche (sogenannte "Spreitschicht"). Dieses Verfahren ist zur Herstellung einer extrem dünnen selbsttragenden Testschicht nicht geeignet. Die filmbildende Masse befindet sich überwiegend auf einer Seite des Gewebes, ist also unsymmetrisch verteilt. Bei der US-4 604 264 wird eine filmbildende Masse, welche Reagenzien enthält, gezielt derartig auf eine Gewebeschicht aufgestrichen, daß sie überwiegend auf einer Seite der Schicht bleibt, also eine unsymmetrische Schichtstruktur resultiert. Dies wird für erforderlich gehalten, um gute optische Eigenschaften der Schicht zu gewährleisten.

Die Faserverbundstruktur besteht vorzugsweise aus monofilen Fäden. Gemäß der Erfindung wird in einem Arbeitsgang ein extrem dünnes Testfeldmaterial mit gleichmäßiger Verteilung der Reagenzfilmmasse erzeugt. In der Praxis wurde beispielsweise ein Testfeldmaterial mit einer Dicke von etwa 0,06 mm hergestellt, welches gemäß Schichtdickenmessung, Inhaltsstoffanalyse und Funktionsprüfung sehr gute Eigenschaften hat. Bei der US-4 604 264 wird dagegen ein monofiles Gewebe als weniger empfehlenswert angesehen und nur in Verbindung mit einer zusätzlichen Trägerfolie verwendet. Dabei werden gleichmäßige und damit für Testzwecke geeignete Eigenschaften erst mit relativ hohen Schichtdicken erreicht. Hieraus resultieren ein hoher Reagenzverbrauch und lange Reaktionszeiten.

Ebenfalls vorbekannt ist es, mit Reagenzien imprägnierte Gewebe oder Vliese als Testfeldmaterialien einzusetzen. Sie unterscheiden sich jedoch sowohl hinsichtlich des Herstellungsverfahrens als auch hinsichtlich ihrer Struktur grundlegend von dem Testfeldmaterial der vorliegenden Erfindung. Die Imprägnierung erfolgt bei den bekannten Verfahren im allgemeinen in der Weise, daß eine Gewebebahn durch einen Imprägniertrog hindurchgeführt wird, in dem sich eine verhältnismäßig dünnflüssige Imprägnierlösung mit den Reagenzien und gegebenenfalls weiteren Hilfsstoffen befindet. Die Bahn wird aus dem Trog herausgeführt und der Überschuß der Imprägnierlösung wird abgestreift, während die Bahn vertikal transportiert wird, so daß der Überschuß in den Trog zurückfließen kann. Das imprägnierte Gewebe behält seine Offenporigkeit. Es handelt sich also nicht um eine geschlossene Schicht, bei der die Gewebeporen von einer relativ hochviskosen Reagenzfilmmasse überspannt werden. Im Rahmen der vorliegenden Erfindung wurde festgestellt, daß die vorbekannte Verfahrensweise selbst dann nicht zu einem befriedigendem Ergebnis führt, wenn die Viskosität der Imprägnierlösung in dem Trog so weit erhöht wird, daß sie die Poren des Gewebes schließt. Vielmehr ergibt sich eine ungleichmäßige Verteilung der Reagenzfilmmasse.

Die Erfindung wird im folgenden anhand eines in den Figuren schematisch dargestellten Ausführungsbeispiels näher erläutert; es zeigen:
- Fig. 1: Eine stark schematisierte Seitenansicht von einer ersten Anlage zur Durchführung des erfindungsgemäßen Verfahrens;
- Fig. 2: eine Detailansicht zu einer abgewandelten Ausführungsform;
- Fig. 3: eine Seitenansicht analog Fig. 1 von einer alternativen Ausführungsform einer Anlage zur Durchführung des erfindungsgemäßen Verfahrens;
- Fig. 4: einen Schnitt durch ein erfindungsgemäßes Testfeldmaterial vor dem Trocknen;
- Fig. 5: einen Schnitt durch einen Testträger mit einem erfindungsgemäßen Testfeldmaterial;
- Fig. 6: eine Aufsicht auf eine für die Erfindung geeignete Faserverbundstruktur.

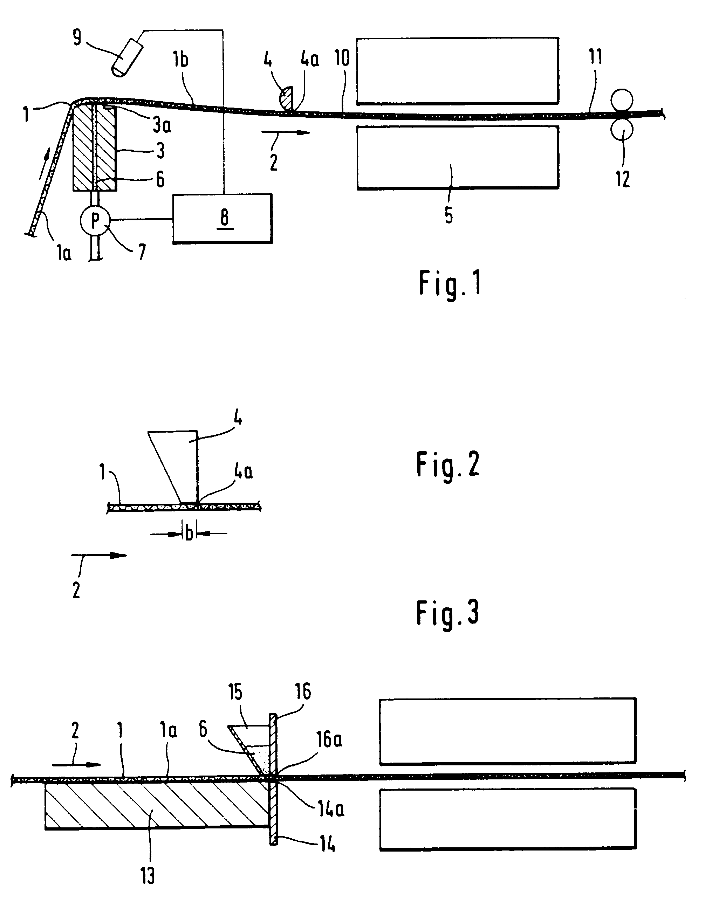

Bei der in Figur 1 dargestellten Anlage wird eine Trägermaterialbahn 1 in Richtung des Pfeiles 2 nacheinander über eine Schlitzdüse 3, unter einem Glattstreichwerkzeug 4 hindurch und durch eine Trocknungskammer 5 geführt.

Die Schlitzdüse 3 und das Glattstreichwerkzeug 4 erstrecken sich mit dem dargestellten Profil (senkrecht zur Zeichenebene) über die gesamte Breite der Trägermaterialbahn 1. Durch die Schlitzdüse 3 wird eine Reagenzfilmmasse 6 derartig in die Trägermaterialbahn gedrückt, daß die Reagenzfilmmasse in die Poren der offenporigen Faserverbundstruktur weitgehend eindringt. Die Auftragsseite 1a ist in diesem Fall die Unterseite der Trägermaterialbahn 1.

Auf der gegenüberliegenden Seite 1b wird die Reagenzfilmmasse mit Hilfe des Glattstreichwerkzeuges 4 derartig glattgestrichen, daß die Poren der Faserverbundstruktur von der Reagenzfilmmasse überspannt werden und auch die Fäden der Trägermaterialbahn von Reagenzfilmmasse (in sehr geringer Dicke) bedeckt sind.

Die Reagenzfilmmasse 6 wird aus einem nicht dargestellten Vorratsbehälter mit Hilfe einer Dosierpumpe 7 zugeführt. Zur Steuerung der Dosierung dient ein Steuergerät 8, welches optional mit einem Ist-Wertgeber 9 verbunden sein kann, der (beispielsweise mit Mitteln der Bildanalyse) das Eindringen der Reagenzfilmmasse 6 in die Trägermaterialbahn 1 beobachtet.

Die exakte Dosierung der Reagenzfilmmasse ist wesentlich für die Qualität des Ergebnisses. Einerseits muß die zugeführte Menge mindestens so groß sein, daß sie weit genug in die offenporige Faserverbundstruktur eindringt, um auf der gegenüberliegenden Seite derartig glattgestrichen werden zu können, daß die Poren der Trägermaterialbahn vollständig geschlossen werden. Andererseits ist aber auch eine Überdosierung nachteilig. Bevorzugt wird die Menge der zugeführten Reagenzfilmmasse derartig beschränkt, daß beim Glattstreichen kein Überschuß abgestreift wird.

In der Praxis hat es sich als ausreichend erwiesen, die Dosierrate, mit der die Reagenzfilmmasse 6 zugeführt wird, empirisch zu bestimmen und das Testfeldmaterial kontinuierlich mit dieser fest eingestellten Dosierrate zu fertigen. Die Möglichkeit einer Regelung mit Hilfe eines Ist-Wertgebers 9, wie sie in Fig. 1 dargestellt ist, ist daher lediglich als zusätzliche Option zu verstehen.

Weiterhin ist wesentlich, daß das nach Passieren des Glattstreichwerkzeuges 4 fertige, jedoch noch feuchte Testfeldmaterial 10 im wesentlichen berührungsfrei getrocknet wird. Es ist vorzugsweise wie dargestellt frei durch die Trockenkammer 5 gespannt. Das getrocknete Testfeldmaterial 11 wird von Transportrollen 12 abgezogen und auf eine nicht dargestellte Rolle aufgewickelt. Selbstverständlich kann die noch feuchte Testfeldmaterialbahn 10 in begrenzten Teilbereichen (insbesondere am Rand) von Führungselementen berührt werden, sofern diese Berührung so gering ist, daß ihre Eigenschaften hierdurch nicht beeinträchtigt werden oder sofern die entsprechenden Teile der getrockneten Testfeldmaterialbahn 11 abgeschnitten und nicht verwendet werden. In diesem Sinne ist "im wesentlichen berührungsfrei" zu verstehen.

Als Glattstreichwerkzeug 4 im Sinne der Erfindung ist jedes Maschinenelement anzusehen, welches sich über die Breite der Trägermaterialbahn 1 erstreckt und geeignet ist, die Reagenzfilmmasse, die von der gegenüberliegenden Seite her in die Faserverbundstruktur eindringt, glattzustreichen. Es ist vorzugsweise - wie dargestellt - so gestaltet, daß es an dem in Transportrichtung 2 hinteren Ende eine Kante 4a aufweist, die die Testfeldmaterialbahn 1 berührt und als Abreißkante dient. Hierdurch wird ein glatter Strich erreicht. Vor der Abreißkante 4a kann die dem Trägermaterial 1 zugewandte Oberfläche des Glattstreichwerkzeuges 4 (wie in Fig. 1 dargestellt) konvex gekrümmt oder (wie in Fig. 2 dargestellt) eben sein. Es wurde gefunden, daß die sogenannte "Rakelbreite", also die Breite b der unteren Fläche (Sohle) des Glattstreichwerkzeuges, die mit dem Trägermaterial 1 in Kontakt steht, relativ gering sein sollte. Bevorzugt liegt sie unter 20 mm, besonders bevorzugt unter 10 mm.

Auch die Schlitzdüse 3 weist vorzugsweise eine Abreißkante 3a auf, um auch auf der Auftragseite la eine glatte Oberfläche zu erzeugen.

Die vorstehende Beschreibung ist nicht so zu verstehen, daß die Reagenzfilmmasse 6 notwendigerweise nur auf einer Seite der Trägermaterialbahn 1 zugeführt wird und nur auf der anderen Seite ein Glattstreichwerkzeug 4 vorgesehen ist. Dies ist zwar im allgemeinen zweckmäßig und ökonomisch. Der Rahmen der Erfindung wird aber nicht verlassen, wenn zusätzlich zu der Zuführung von einer ersten Seite und zu dem Glattstreichen von einer zweiten Seite des Trägermaterials eine Zuführung auch von der zweiten Seite her und unter Umständen ein Glattstreichen von der ersten Seite her erfolgt.

Ein extrem dünnes selbsttragendes Testfeldmaterial hat besondere praktische Bedeutung im Zusammenhang mit Testträgern, bei denen die Probe (insbesondere Vollblut) auf die eine Seite des Testfeldmaterials aufgegeben und eine resultierende Farbreaktion auf der anderen Seite des Testfeldmaterials beobachtet wird. Die Reagenzfilmmasse enthält vorzugsweise ein Farbbildungsreagenz und ein Pigment. Im Hinblick auf eine für derartige Anwendungszwecke erforderliche ausreichende Durchlässigkeit für die Probenflüssigkeit ist es vorteilhaft, wenn ein Dispersionsfilmbildner (statt eines gelösten Filmbildners) verwendet wird.

Nähere Angaben über bevorzugte Reagenzfilmmassen und die damit erzielten Vorteile sind der deutschen Patentanmeldung P 41 06 293.0 zu entnehmen, auf welche Bezug genommen wird.

Bei der in Figur 3 dargestellten Ausführungsform erfolgt die Zuführung der Reagenzfilmmasse 6 von oben. Die Trägermaterialbahn 1 wird in Richtung des Pfeiles 2 über einen Rakelstein 13 geführt. Nahe dem Ende des Rakelsteins (bezogen auf die Transportrichtung 2) befindet sich oberhalb der Materialbahn 1 ein Rakelkasten 15, der in Transportrichtung von einer Rakel (gebräuchlich ist auch die Bezeichnung "Rakelmesser") abgeschlossen und mit Reagenzfilmmasse 6 gefüllt ist. In diesem Fall ist die Oberseite der Trägermaterialbahn 1 die Auftragsseite 1a und kontaktiert die untere Fläche (Rakelfläche) der Rakel 16 von unten. Als Glattstreichwerkzeug dient dabei vorzugsweise - wie dargestellt - eine zweite Rakel 14, über die die Trägermaterialbahn 1 geführt wird (so daß die Rakelfläche der Rakel 14 die Trägermaterialbahn von unten kontaktiert). Dabei soll vorzugsweise die Abreißkante 14a der Rakel 14 in Transportrichtung 2 bündig mit der Abreißkante 16a der Rakel 16 verlaufen.

Gemäß einer alternativen jedoch gegenüber Figur 3 weniger bevorzugten Ausführungsform kann auch der Rakelstein 13 als Glattstreichwerkzeug dienen. In diesem Fall sollte seine in Transportrichtung 2 hintere Kante, über die die Trägermaterialbahn 1 läuft, sich in Transportrichtung kurz hinter dem Rakelkasten 15 befinden und vorzugsweise bündig mit der Abreißkante 16a der Rakel 16 verlaufen.

Durch diese Anordnung wird eine Selbstdosierung erreicht, wobei die Dosierrate, mit der die Reagenzfilmmasse 6 in die Trägermaterialbahn 1 eingebracht wird, durch die Größe des Rakelspaltes, also den Abstand der Unterkante des Rakellineals 16 von dem Rakelstein 14, einstellbar ist. Da das Volumen des Rakelkastens 15 auf der Auslaufseite von dem Rakellineal 16 begrenzt ist, verbleibt der Überschuß an Reagenzfilmmasse, der von dem Rakellineal abgestreift wird, im Rakelkasten 15.

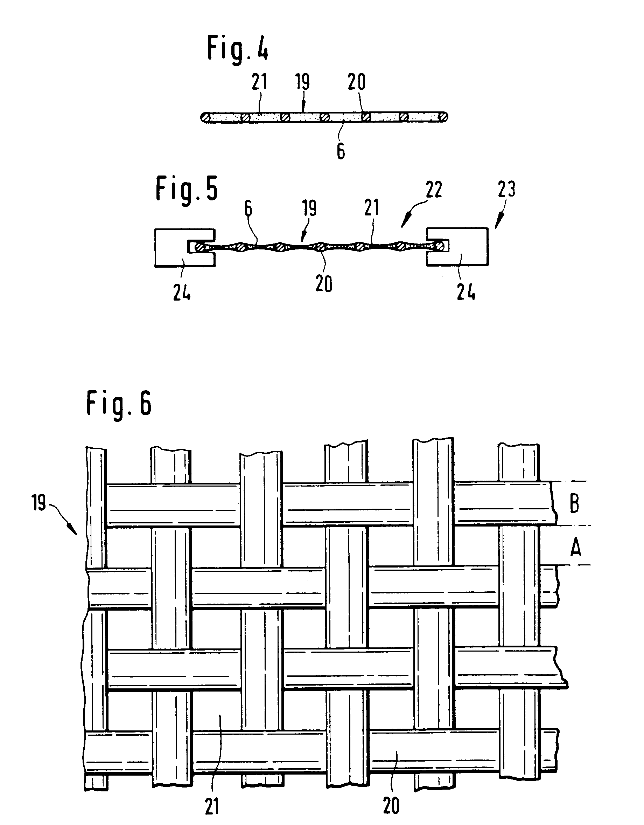

Die Figuren 4 und 5 zeigen im Querschnitt ein Testfeldmaterial, bei dem eine Reagenzfilmmasse 6 in eine Faserverbundstruktur 19 eingebettet ist. Die Fäden 20 der Faserverbundstruktur 19 haben vorzugsweise eine geringe Dicke (maximal 0,1 mm, bevorzugt 0,02 mm bis 0,06 mm) und sind vorzugsweise monofil.

Die Reagenzfilmmasse 6 ist so aufgetragen, daß die Poren 21 der Faserverbundstruktur 19 praktisch vollständig gefüllt und die Fäden 20 beidseitig dünn mit Reagenzfilmmasse bedeckt sind. Der Zustand der feuchten Trägermaterialbahn 10 nach dem Glattstreichen ist in Fig. 4 stark schematisiert dargestellt.

Durch den Trocknungsvorgang verringert sich die Schichtdicke der Reagenzfilmmasse. Dadurch entsteht eine charakteristische Struktur, bei der die Oberfläche der Testschicht zwischen den Fäden leicht konkav gekrümmt ist, wie dies in Fig. 2 angedeutet ist.

Vorzugsweise wird das erfindungsgemäße Testfeldmaterial ohne irgendwelche weiteren Testschichten als selbsttragendes Testfeld verwendet. Einen solchen Testträger 23 zeigt Fig. 5, wobei das Testfeld 22 von einem Rahmen 24 eingefaßt ist.

Figur 6 zeigt eine typische Struktur eines für die Erfindung geeigneten monofilen Gewebes. Die Fäden haben ein lockere gitterförmige Anordnung mit offenen Poren 21. Die Porengröße A sollte zwischen 0,02 und 0,08 mm, vorzugsweise zwischen 0,03 und 0,05 mm liegen. Im dargestellten Fall quadratischer Poren bezeichnet der Begriff "Porengröße" deren Kantenlänge. Bei einer von der quadratischen Form abweichenden Porenform ist die Porengröße als Quadratwurzel aus dem mittleren Porenquerschnitt definiert.

Die Dicke B der Fäden, aus denen die Faserverbundstruktur gebildet ist, liegt wie erwähnt vorzugsweise zwischen 0,02 mm und 0,06 mm, wobei Werte zwischen 0,03 mm und 0,04 mm besonders bevorzugt sind. Als Fadenmaterial hat sich beispielsweise Polyester bewährt. Es sind jedoch auch andere Materialien geeignet.

## Patentansprüche

1. Verfahren zur Herstellung eines selbsttragenden Testfeldmaterials mit einer Schichtdicke von weniger als 0,1 mm zur Bestimmung eines Analyten in einer Körperflüssigkeit, bei welchem
(a) eine Reagenzfilmmasse (6) in eine aus einer offenporigen Faserverbundstruktur bestehende Trägermaterialbahn (1) von einer Auftragsseite (1a) her derartig gepreßt wird, daß sie in die Poren des Trägermaterials weitgehend eindringt,
(b) die Reagenzfilmmasse (6) auf der gegenüberliegenden Seite (1b) durch die Relativbewegung der Bahn und eines Glattstreichwerkzeuges (4,14) derartig glattgestrichen wird, daß die Poren (21) der Faserverbundstruktur von der Reagenzfilmmasse (6) überspannt werden, und
(c) die so erzeugte Testfeldmaterialbahn (10) im wesentlichen berührungsfrei getrocknet wird.

2. Verfahren nach Anspruch 1, bei welchem die Reagenzfilmmasse (6) derartig dosiert zugeführt wird, daß bei dem Glattstreichen an dem Glattstreichwerkzeug (4,14) praktisch kein Überschuß abgestreift wird.

3. Verfahren nach einem der vorhergehenden Ansprüche, bei welchem die Faserverbundstruktur (19) ein einlagiges Gewebe oder Gewirke ist, welches vorzugsweise aus monofilen Fäden (20) besteht.

4. Verfahren nach einem der vorhergehenden Ansprüche, bei welchem die Viskosität der Reagenzfilmmasse (6) beim Hineinpressen in das Trägermaterial (1) zwischen 10 mPasec und 300 mPasec, bevorzugt zwischen 20 mPasec und 200 mPasec beträgt.

5. Verfahren nach einem der vorhergehenden Ansprüche, bei welchem die Reagenzfilmmasse ein Farbbildungsreagenz und ein Pigment enthält.

6. Verfahren nach einem der vorhergehenden Ansprüche, bei welchem die Reagenzfilmmasse (6) einen Dispersionsfilmbildner enthält.

7. Verfahren nach einem der vorhergehenden Ansprüche, bei welchem das Glattstreichwerkzeug (4,14) an seinem in Transportrichtung der Trägermaterialbahn hinteren Ende eine Kante aufweist.

8. Verfahren nach einem der vorhergehenden Ansprüche, bei welchem die Reagenzfilmmasse von oben aus einem Rakelkasten (15) auf die Trägermaterialbahn (1) aufgerakelt wird, wobei der Rakelkasten (15) in Transportrichtung (2) von einer die Trägermaterialbahn (1) von oben kontaktierenden Rakel (16) abgeschlossen wird, und die Trägermaterialbahn (1) über ein sie von unten kontaktierendes Glattstreichwerkzeug (14) läuft.

9. Verfahren nach Anspruch 8, bei welchem das Glattstreichwerkzeug (14) eine Abreißkante (14a) hat, die in Transportrichtung (2) bündig mit der Abreißkante (16a) der Rakel (16) verläuft.

10. Verfahren nach einem der Ansprüche 1 bis 7, bei welchem die Trägermaterialbahn (1) über eine Schlitzdüse (3) geführt, die Reagenzfilmmasse (6) von unten durch die Schlitzdüse (3) in das Trägermaterial (1) gepreßt und danach glattgestrichen wird.

11. Testfeldmaterial mit einer Schichtdicke von weniger als 0,1 mm für einen Testträger zur Bestimmung eines Analyten in einer Körperflüssigkeit, bei welchem ein Reagenzfilmmaterial in einer offenporigen Faserverbundstruktur (19) derartig eingebettet ist, daß es deren Poren (21) überspannt und damit schließt, wobei das Reagenzfilmmaterial aus einer filmbildenden zähflüssigen Reagenzfilmmasse gebildet ist, welche in die Faserverbundstruktur eingepreßt und beidseitig glattgestrichen ist.

12. Testfeldmaterial nach Anspruch 11, bei welchem die mittlere Dicke weniger als 0,07 mm beträgt.

13. Testfeldmaterial nach Anspruch 11 oder 12, bei welchem die mittlere Porengröße der Faserverbundstruktur zwischen 0,02 mm und 0,08 mm, vorzugsweise zwischen 0,03 und 0,05 mm beträgt.

## Revendications

1. Procédé de fabrication d'un matériau autoporteur pour une zone de test avec une épaisseur de couche inférieure à 0,1 mm pour la détermination d'un analyte dans un liquide corporel, dans lequel
(a) une masse pour film réactif (6) est directement pressée d'un coté appliqué (1a) dans une bande de matériau support (1) constituée d'une structure de fibres à pores ouverts, de sorte qu'elle pénètre largement dans les pores du matériau support,
(b) la masse pour film réactif (6) est lissée sur le côté opposé (1b) à l'aide du mouvement relatif de la bande et d'un outil de lissage (4,14) de telle sorte que les pores (21) de la structure de fibres sont recouverts de la masse pour film réactif (6), et
(c) la bande de matériau pour zone de test (10) ainsi fabriquée est séchée essentiellement sans contact.

2. Procédé selon la revendication 1, dans lequel la masse pour film réactif (6) introduite est dosée de telle sorte que lors du lissage par l'outil de lissage (4,14) il ne lui est retiré aucun excédent.

3. Procédé selon l'une quelconque des revendications précédentes, dans lequel la structure de fibres (19) est un tissu ou un tricot monocouche, lequel est constitué de préférence de fibres monofilamentaires (20).

4. Procédé selon l'une quelconque des revendications précédentes, dans lequel lors du pressage dans le matériau support (1) la viscosité de la masse pour film réactif (6) est comprise entre 10 mPa.s et 300 mPa.s, de préférence entre 20 mPa.s et 200 mPa.s.

5. Procédé selon l'une quelconque des revendications précédentes, dans lequel la masse pour film réactif (6) contient un réactif de coloration et un pigment.

6. Procédé selon l'une quelconque des revendications précédentes, dans lequel la masse pour film réactif (6) contient un agent filmogène de dispersion.

7. Procédé selon l'une quelconque des revendications précédentes, dans lequel l'outil de lissage (4,14) présente une arête au niveau de son extrémité arrière dans le sens du transport de la bande de matériau support.

8. Procédé selon l'une quelconque des revendications précédentes, dans lequel la masse pour film réactif (6) est couchée sur la bande de matériau support (1) à l'aide d'un racloir à partir d'une trémie de racloir (15) au-dessus de celle-ci, dans lequel la trémie de racloir (15) est terminée par un racloir (16) faisant contact par le haut avec la bande de matériau support (1) dans la direction du transport (2), et la bande de matériau support (1) passe par-dessus un outil de lissage (14) en contact avec elle par dessous.

9. Procédé selon la revendication 8, dans lequel l'outil de lissage (14) possède un bord d'arrachage (14a), qui se prolonge au même niveau que le bord d'arrachage (16a) du racloir (16) dans la direction du transport (2).

10. Procédé selon l'une quelconque des revendications 1 à 7, dans lequel la bande de matériau support (1) est dirigée au-dessus d'une buse à fente (3), la masse pour film réactif (6) provenant du bas à travers la buse à fente (3) est pressée dans le matériau support (1) puis lissée.

11. Matériau pour zone de test possédant une épaisseur de couche inférieure à 0,1 mm pour un support de test pour la détermination d'un analyte dans un liquide corporel, dans lequel un matériau pour film réactif est incorporé dans une structure de fibres à pores ouverts (19) de telle sorte qu'il en recouvre les pores (21) et ainsi les ferme, dans lequel le matériau pour film réactif est formé d'une masse pour film réactif filmogène et visqueuse, laquelle est incorporée par pressage dans la structure de fibres et lissée des deux côtés.

12. Matériau pour zone de test selon la revendication 11, dans lequel l'épaisseur moyenne est inférieure à 0,07 mm.

13. Matériau pour zone de test selon la revendication 11 ou 12, dans lequel la taille moyenne de pores de la structure de fibres est comprise entre 0,02 mm et 0,08 mm, de préférence entre 0,03 mm et 0,05 mm.

## Claims

1. Method for the manufacture of a self-supporting test field material for the determination of an analyte in a body fluid having a layer thickness of less than 0.1 mm, in which:
(a) A reagent film mass (6) is pressed from an application side (1a) into a carrier material band (1) consisting of an open-pored fibre structure in such a way that it penetrates to a large extent into the pores of the carrier material,
(b) the reagent film mass (6) on the opposite side (1b) is smoothed by the relative motion of the band and a smoothing tool (4, 14) in such a way that the pores (21) of the fibre structure are spanned by the reagent film mass (6), and
(c) the strip of test field material (10) thus produced is dried essentially without contact.

2. Method according to claim 1 in which the reagent film mass (6) is fed metered in such a way that during smoothing, practically no excess is wiped off by the smoothing tool (4, 14).

3. Method according to any one of the preceding claims, in which the fibre structure (19) is a single-layer woven or knitted fabric which preferably consists of monofilamentous threads (20).

4. Method according to any one of the preceding claims, in which the viscosity of the reagent film mass (6) during pressing into the carrier material (1) is between 10 mPa.s and 300 mPa.s, preferably between 20 mPa.s and 200 mPa.s.

5. Method according to any one of the preceding claims, in which the reagent film mass contains a colour-forming reagent and a pigment.

6. Method according to any one of the preceding claims, in which the reagent film mass (6) contains a dispersion film former.

7. Method according to any one of the preceding claims, in which the smoothing tool (4, 14) has an edge at its end rearward in the transport direction of the carrier material band.

8. Method according to any one of the preceding claims, in which the reagent film mass is provided from above by means of a doctor box (15) onto the carrier material band (1), the doctor box (15) being closed in the transport direction (2) by a doctor (16) contacting the carrier material band (1) from above, and the carrier material band (1) travels over a smoothing tool (14) contacting it from below.

9. Method according to claim 8, in which the smoothing tool (14) has a sharp edge (14a) which in the transport direction (2) follows a course flush with the sharp edge (16a) of the doctor (16).

10. Method according to any one of claims 1 to 7, in which the carrier material band (1) is guided over a slit orifice (3), the reagent film mass (6) is pressed from below through the slit orifice (3) into the carrier material (1) and thereafter smoothed.

11. Test field material for a test carrier for the determination of an analyte in a body fluid having a layer thickness of less than 0.1 mm, in which a reagent film mass is embedded in an open-pored fibre structure (19) in such a way that it spans and thus closes its pores (21), the reagent film mass being formed from a film-forming, viscous reagent film mass which is pressed into the fibre structure and smoothed on both sides.

12. Test field material according to claim 11, in which the mean thickness is less than 0.07 mm.

13. Test field material according to claim 11 or 12, in which the average pore size of the fibre structure is between 0.02 mm and 0.08 mm, preferably between 0.03 and 0.05 mm.
